# EUROPEAN PATENT SPECIFICATION

(11) **EP 3 898 986 B1**
(45) Date of publication and mention of the grant of the patent: **18.02.2026**
(21) Application number: 19813528.7
(22) Date of filing: 04.12.2019
(51) Int. Cl.: C12N 15/82, C07K 14/415, A01H 5/12, A01H 6/14, A01H 1/04, C12Q 1/6895

(54) **LETTUCE PLANT RESISTANT TO DOWNY MILDEW AND RESISTANCE GENE**
GEGEN FALSCHEN MEHLTAU RESISTENTE SALATPFLANZE UND RESISTENZGEN
PLANTE DE LAITUE RÉSISTANT AU MILDIOU ET GÈNE DE RÉSISTANCE

(30) Priority: 17.12.2018 WO PCT/EP2018/085244
(43) Date of publication of application: 27.10.2021
(73) Proprietor: Enza Zaden Beheer B.V., 1602 DB Enkhuizen (NL)
(72) Inventor: TER RIET, Bas, 1602 DB Enkhuizen (NL); PEL, Mathieu André, 1602 DB Enkhuizen (NL); BROOS, Stephanie Melanie, 1602 DB Enkhuizen (NL)
(74) Representative: Arnold & Siedsma
(86) International application number: PCT/EP2019/083651
(87) International publication number: WO 2020/126500

(56) References cited:
- WO-A1-2015/136085
- WO-A1-98/30083
- DATABASE GenBank - Nucleotide [online] NCBI; 6 February 2018 (2018-02-06), "PREDICTED: Lactuca sativa putative disease resistance protein At3g14460 (LOC111918844), partial mRNA.", XP002793007, Database accession no. XM_023914461
- DATABASE EMBL [online] 15 January 2018 (2018-01-15), "Lactuca sativa hypothetical protein", XP002793008, Database accession no. PLY80533

## Description

The present invention relates to a lettuce plant that is resistant to downy mildew, more specifically to a lettuce plant that comprises a mutated gene that confers broad spectrum resistance to *Bremia lactucae* in lettuce. Furthermore the present invention relates a resistance gene and a method for obtaining a lettuce plant that is resistant to downy mildew, wherein the method comprises the step of mutating a gene.

Downy mildew refers to several types of oomycete microbes that are parasites of plants. Downy mildew can originate from various species, but mainly of Peronospora, Plasmopara and Bremia. Downy mildew is a problem in many food crops, in for example in lettuce caused by *Bremia lactucae,* affecting the production of this crop worldwide. Plants that are being affected include food crops such as brassicas (e.g. cabbage), potatoes, grape, spinach, lettuce, onion, tomato, cucumber plants. Downy mildew infection show symptoms of discoloured areas on upper leaf surfaces in combination with white, grey or purple mould located on the other side of the leaf surface below. Disease is spread from plant to plant by airborne spores.

Lettuce, mostly known as *Lactuca sativa,* but also including Lactuca species such as *L. serriola, L. saligna or L. virosa,* is a very important crop worldwide. Some of the most popular varieties available are Iceberg, Romaine, Butterhead, Batavia and Oakleaf. There are many plant pathogens that affect *L. sativa,* and some of the diseases caused by these pathogens are downy mildew, sclerotinia rot, powdery mildew, fusarium wilt of which the most important disease is lettuce downy mildew, which is caused by the *B. lactucae,* an oomycete pathogen that belong to *Peronosporaceae.*

For some vegetable crops, such as lettuce, cultivars with resistance to downy mildew are available. However, the pathogen under pressure will mutate to break down the disease resistance and new disease resistance in crops is needed to control infection. Especially in lettuce the occurrence of resistant downy mildew is particularly complex as there are many different races, and new resistant downy mildew species emerging all the time.

In lettuce, infection of *B. lactucae* result in yellow to pale green lesions that eventually become necrotic due to secondary pathogens leading to major crop losses. Fungicides can be used to control *B. lactucae,* but eventually *B. lactucae* becomes immune to these chemicals, because over time the pathogen also acquires resistance to fungicides. Furthermore, there are multiple lettuce varieties available that are resistant to *B. lactucae* but resistance is quickly overcome because new Bremia races develop rapidly. Therefore, it is of the utmost importance to find other methods to control *B. lactucae* infection. Most preferably is to identify a resistance gene that gives broad resistance against *B. lactucae* and to provide for lettuce plants that are resistant to downy mildew. Therefore, identification of resistance genes is a promising alternative.

Considering the above, there is a need in the art for to provide plants that are resistant to downy mildew and wherein plants have a broad spectrum resistance against this pathogen. Furthermore, it is an object of present invention to provide a method to obtain such downy mildew resistant plants.

It is an object of the present invention, amongst other objects, to address the above need in the art. The object of present invention, amongst other objects, is met by the present invention as outlined in the appended claims.

Specifically, the above object, amongst other objects, is met, according to a first aspect, by the present invention by a lettuce plant that is resistant to downy mildew, wherein said plant comprises one or more mutations in a *SL7* gene, wherein said *SL7* gene encodes for a protein sequence of SEQ ID No. 2 or having at least 90% sequence identity with SEQ ID No. 2, preferably at least 95%, more preferably at least 98%, even more preferably at least 99%, most preferably 100%, wherein the one or more mutations in the SL7 gene result in amino acid substitutions on position 38 in the SL7 protein represented by SEQ ID No.2. Preferably the serine (S) at position 38 in SEQ ID No. 2 is mutated to asparagine (N). The mutated *SL7* gene is a dominant resistance gene, and may be homozygous or heterozygous present in a downy mildew resistant lettuce plant.

The majority of disease resistance genes in plants encode nucleotide-binding site leucine-rich repeat proteins, also known as NBS-LRR proteins (encoded by R genes). These proteins are characterized by nucleotide-binding site (NBS) and leucine-rich repeat (LRR) domains as well as variable amino- and carboxy-terminal domains and are involved in the detection of diverse pathogens, including bacteria, viruses, fungi, nematodes, insects and oomycetes. There are two major subfamilies of plant NBS-LRR proteins defined by the Toll/interleukin-1 receptor (TIR) or the coiled-coil (CC) motifs in the amino-terminal domain and are both involved in pathogen recognition.

A leucine-rich repeat (LRR) is a protein structural domain composed of repeating 20 to 30 amino acid stretches that forms an α/β horseshoe fold. The domain is rich in the hydrophobic amino acid leucine. The region between the helices and sheets is the protein's hydrophobic core and is tightly sterically packed with leucine residues. On average classical NBS-LLR genes comprise six LRR regions. The SL7 gene is not a classical NBS-LRR gene, since the SL7 protein does not comprise multiple LRR regions, and it does not comprise the NBS domain. The SL7 gene contains only one LRR region, in which the SL7 gene differs from other cases of R genes where multiple LRR regions and the NBS domain are present. It is thought that those domains determine effector recognition and therefore disease susceptibility/resistance. The presence of the SL7 resistance gene will decrease the chances of the pathogen overcoming the resistance, as often seen with the R genes. Even so, combined with R genes, disease resistance (e.g. against downy mildew) may even be further improved.

For the first time a resistance gene has been found in a lettuce plant that is located on chromosome 3 and that can be linked to plant disease resistance. This *SL7* gene of present invention gives resistance to *Bremia lactucae* races B117 to B135, with the exception of B116, B120, B121, B123, and B127. Preferably, spectrum resistance to *Bremia lactucae* in lettuce comprises resistance to *Bremia lactucae* of at least races B117, B118, B122, B124 to B126, and B128 to B135.

To demonstrate that the SL7 gene is related to Bremia resistance, this putative resistance gene has been silenced by tobacco rattle virus (TRV)-based virus-induced gene silencing (VIGS) to induce susceptibility to *B. lactucae* infection in resistant *L. saligna* lettuce lines containing the SL7 resistance gene. With VIGS it was demonstrated that the *SL7* gene was associated with downy mildew resistance, VIGS gene silencing was used to create Bremia-susceptibility in resistant Lactuca species. Resistant lettuce plants were transient transformed with an *SL7* silencing construct and made susceptible to *B. lactucae* infection, thus by removing the *SL7* gene via virus induced gene silencing. According to another preferred embodiment, the present invention relates to the Lettuce plant, wherein the one or more mutations in the *SL7* gene result in amino acid substitutions in the region comprised of amino acid positions 6 to 147 in the SL7 protein represented by SEQ ID No.2. The region comprised of amino acids 6 to 147 is an LRR region of SL7, comprising at least one or more LLR domains, preferably at least two, more preferably at least three, most preferably four LLR domains.

According to yet another preferred embodiment, the present invention relates to the Lettuce plant, wherein the one or more mutations in the *SL7* gene further result in amino acid substitutions on position 11 in the SL7 protein represented by SEQ ID No.2. Preferably the threonine (T) at position 11 in SEQ ID No. 2 is mutated to arginine (R).

According to another preferred embodiment, the present invention relates to the Lettuce plant, wherein the one or more mutations in the *SL7* gene result in amino acid substitutions in the region comprised of amino acid positions 26 to 72 in the SL7 protein represented by SEQ ID No.2. The region comprised of amino acids 26 to 72 is a single LRR domain of SL7.

According to yet another preferred embodiment, the present invention relates to the Lettuce plant, wherein the one or more mutations in the *SL7* gene further result in amino acid substitutions on position 40 in the SL7 protein represented by SEQ ID No.2. Preferably the lysine (K) at position 40 in SEQ ID No. 2 is mutated to threonine (T).

According to yet another preferred embodiment, the present invention relates to the Lettuce plant, wherein the one or more mutations in the *SL7* gene further result in amino acid substitutions on position 48 in the SL7 protein represented by SEQ ID No.2. Preferably the serine (S) at position 48 in SEQ ID No. 2 is mutated to asparagine (N).

According to yet another preferred embodiment, the present invention relates to the Lettuce plant, wherein the one or more mutations in the *SL7* gene further result in amino acid substitutions on position 61 in the SL7 protein represented by SEQ ID No.2. Preferably the threonine (T) at position 61 in SEQ ID No. 2 is mutated to serine (S).

According to yet another preferred embodiment, the present invention relates to the Lettuce plant, wherein the one or more mutations in the *SL7* gene further result in amino acid substitutions on position 69 in the SL7 protein represented by SEQ ID No.2. Preferably the isoleucine (I) at position 69 in SEQ ID No. 2 is mutated to valine (V).

According to yet another preferred embodiment, the present invention relates to the Lettuce plant, wherein the one or more mutations in the *SL7* gene further result in amino acid substitutions on position 84 in the SL7 protein represented by SEQ ID No.2. Preferably the cysteine (C) at position 84 in SEQ ID No. 2 is mutated to arginine (R).

According to yet another preferred embodiment, the present invention relates to the Lettuce plant, wherein the one or more mutations in the *SL7* gene further result in amino acid substitutions on position 91 in the SL7 protein represented by SEQ ID No.2. Preferably the aspartic acid (D) at position 91 in SEQ ID No. 2 is mutated to asparagine (N).

According to yet another preferred embodiment, the present invention relates to the Lettuce plant, wherein the one or more mutations in the *SL7* gene further result in amino acid substitutions on position 129 in the SL7 protein represented by SEQ ID No.2. Preferably the valine (V) at position 129 in SEQ ID No. 2 is mutated to isoleucine (I).

According to another preferred embodiment, the present invention relates to the Lettuce plant, wherein the mutations in the SL7 gene result in amino acid substitutions at position 11, 38, 40, 48, 61, 69, 84, 91 and 129 in the SL7 protein represented by SEQ ID No.2. Preferably the mutations are T11R, S38N, K40T, S48N, T61S, I69V, C84R, D91N, and V129I respectively. An *SL7* resistance gene of present invention that encodes for the protein that comprises all the above mutations is represented by SEQ ID No. 3 and encodes for the SL7 protein is represented by SEQ ID No. 4.

According to another preferred embodiment, the present invention relates to the Lettuce plant, wherein the mutations in the SL7 gene further result in amino acid substitutions at position 11, 40, and 84 in the SL7 protein represented by SEQ ID No.2. Preferably the mutations are T11R, S38N, K40T, and C84R.

According to another preferred embodiment, the present invention relates to the Lettuce plant, wherein the SL7 gene that comprises one or more mutations and encodes for the protein sequence represented by SEQ ID No. 4. The mutated SL7 gene, being the SL7R gene, is represented by SEQ ID No. 3. Sequencing experiments showed that the protein encoded by the *SL7R* gene from the resistant plant compared with the protein encoded by the *SL7* gene of a plant that is susceptible differs in several amino acids that have been mutated. In particular mutations in the LRR region of the SL7 protein in the amino acid 6 to 147 are of importance to provide resistance to downy mildew, more specifically in the LLR domain of the SL7 protein in the amino acid region of 26 to 72. The downy mildew is caused in the Lettuce plant by an oomycete, more preferably *Bremia lactucae.*

According to yet another preferred embodiment, the present invention relates to the lettuce plant, wherein the plant is selected from *Lactuca sativa, Lactuca virosa, Lactuca saligna, Lactuca serriola, Lactuca aculeate, Lactuca georgica, Lactuca perennis, Lactuca tatarica, Lactuca viminea,* preferably *Lactuca sativa.*

According to a preferred embodiment, the present invention relates to the lettuce plant, wherein the mutations in the *SL7* gene are obtainable by gene editing techniques, preferably by mutagenesis (e.g. EMS) and/or CRISPR/Cas.

According to another preferred embodiment, the present invention relates to the lettuce plant, wherein the lettuce plant is resistant to downy mildew caused by one or more of *Bremia lactucae* selected from the group of race B117, B118, B122, B124 to B126, B128 to B135. A lettuce plant of present invention comprising the SL7 resistance gene is susceptible to downy mildew caused by *Bremia lactucae* B116, B120, B121, B123, and B127. Preferably, spectrum resistance to *Bremia lactucae* in the lettuce of present invention comprises resistance to *Bremia lactucae* of at least races B117, B118, B122, B124 to B126, B128 to B135.

According to yet another preferred embodiment, the present invention relates to the lettuce plant, wherein the resistance gene *SL7R* of SEQ ID No. 3 is obtainable from deposit number NCIMB 42785.

The present invention, according to a second aspect, relates to seeds produced by the lettuce plant of present invention. The seed comprises the SL7R gene as described above.

The present invention, according to a third aspect, relates to a resistance gene *SL7R* that confers resistance to *Bremia lactucae* in lettuce plants, wherein the gene comprises a coding sequence of SEQ ID No. 3 or having at least 90% sequence identity with SEQ ID No. 3, preferably at least 95%, more preferably at least 98%, most preferably at least 99%, most preferably 100%. The *SL7R* gene is a dominant gene. SEQ ID No.3 represents the coding nucleotide sequence of *SL7R* gene of *Lactuca saligna* and encodes for the SL7R protein sequence represented by SEQ ID No.4. SEQ ID No.4 represents the SL7R protein sequence of *Lactuca saligna* and lettuce plants that express this protein show complete resistance to downy mildew.

According to a preferred embodiment, the present invention relates to resistance gene *SL7R,* wherein the gene encodes for a SL7R protein that has at least 85% sequence identity with SEQ ID No. 4, preferably at least 90%, more preferably at least 95%, most preferably at least 98%, most preferably 100%.

According to another preferred embodiment, the present invention relates to the resistance gene *SL7R,* wherein resistance to *Bremia lactucae* in lettuce comprises resistance to *Bremia lactucae* of race B117, B118, B122, B124 to B126, B128 to B135. Preferably, spectrum resistance to *Bremia lactucae* in lettuce comprises resistance to *Bremia lactucae* of at least races B117, B118, B122, B124 to B126, B128 to B135.

According to yet another preferred embodiment, the present invention relates to the resistance gene *SL7R,* wherein the plant is selected from *Lactuca sativa, Lactuca virosa, Lactuca saligna, Lactuca serriola, Lactuca aculeate, Lactuca georgica, Lactuca perennis, Lactuca tatarica, Lactuca viminea,* preferably *Lactuca sativa.*

Disclosed but not part of the claimed invention is a method for obtaining a lettuce plant that is resistant to downy mildew, wherein the method comprises the steps of,
a) crossing a lettuce plant comprised of the resistance gene *SL7R* of present invention with a lettuce plant that does not comprise said SL7R gene,
b) optionally, selfing the plant obtained in step a) for at least one time,
c) selecting the plants that are resistant to downy mildew.
In the method of present invention the lettuce plant is selected from *Lactuca sativa, Lactuca virosa, Lactuca saligna, Lactuca serriola, Lactuca aculeate, Lactuca georgica, Lactuca perennis, Lactuca tatarica, Lactuca viminea,* preferably *Lactuca sativa.*

The present invention, according to a further aspect, relates to a method for obtaining a lettuce plant that is resistant to downy mildew, wherein the method comprises the step of providing one or more mutations in a *SL7* gene of a lettuce plant, resulting in a *SL7R* resistance gene of present invention. The *SL7* gene comprises a coding sequence that has at least 90% sequence identity with SEQ ID No. 1, preferably at least 95%, more preferably at least 98%, most preferably at least 99%, most preferably 100%. SEQ ID No.1 represents the coding nucleotide sequence of the *SL7* gene of *Lactuca sativa.* This sequence is the wild type sequence and does not contain the mutations as compared to the resistance gene of present invention.

According to another preferred embodiment, the present invention relates to the method, wherein the one or more mutations in the *SL7* gene result in amino acid substitutions in the region comprised of amino acid positions 6 to 147, preferably comprised of amino acid positions 26 to 72, in the SL7 protein represented by SEQ ID No.2. Mutations are located in the LLR region of amino acid positions 6 to 147, preferably in the single LRR domain that is located from amino acid 26 to 72 of the SL7 protein.

According to yet another preferred embodiment, the present invention relates to the method, wherein the one or more mutations in the SL7 gene comprise amino acid substitutions at position 11, 38, 40, and 84 in the SL7 protein represented by SEQ ID No.2.

According to yet another preferred embodiment, the present invention relates to the method, wherein the one or more mutations in the SL7 gene further comprises amino acid substitutions at position 48, 61, 69, 91 and 129 in the SL7 protein represented by SEQ ID No.2.

According to a preferred embodiment, the present invention relates to the method, wherein the *SL7* gene that comprises one or more mutations, being the *SL7R* gene, is represented by SEQ ID No. 3 and encodes for the protein sequence represented by SEQ ID No. 4. SEQ ID No.4 represents the *SL7R* protein sequence of *Lactuca saligna.* Lettuce, such as *L. sativa* that express the protein of SEQ ID No.4 is resistant to downy mildew.

According to a preferred embodiment, the present invention relates to the method, wherein the mutations in the *SL7* gene are obtained by gene editing techniques, preferably by mutagenesis or CRISPR/Cas. The lettuce plant comprising the mutations in the SL7 gene is selected from *Lactuca sativa, Lactuca virosa, Lactuca saligna, Lactuca serriola, Lactuca aculeate, Lactuca georgica, Lactuca perennis, Lactuca tatarica, Lactuca viminea,* preferably *Lactuca sativa.* A lettuce plant comprised of the mutations in the SL7 gene gives a high downy mildew resistance phenotype. A plant having this resistant phenotype can be obtained via use of gene editing and/or mutation techniques, such as EMS mutagenesis or CRISPR/Cas in concert with cloning techniques on the *SL7* gene to generate disease resistant crops. Mutations induced by gene editing techniques such as mutagenesis, CRISPR/Cas, transgenic techniques, or others can be regarded as non-natural mutations. Alternatively, a *SL7R* gene can be brought into the plant by means of transgenic techniques or by introgression.

The present invention, according to a further aspect, relates to the use of a gene construct for introducing a resistance gene into the genome of a plant or plant cell, wherein the gene construct is comprised of the resistance gene SL7R of present invention which is operably linked to expression providing sequences in said plant. The resistance gene of present invention may be transferred (e.g. by transformation or transfection) into plants, such as lettuce plants, using a plasmid of vector or linear gene construct that comprises the *SL7R* resistance gene of present invention or wherein the gene comprises a coding sequence that has at least 90% sequence identity with SEQ ID No. 3. The resistance gene *SL7R* encodes for a SL7R protein that has at least 85% sequence identity with SEQ ID No. 4. The Resistance gene *SL7R,* after being transferred into the lettuce plant would provide resistance to *Bremia lactucae* , i.e. resistance to *Bremia lactucae* of race B117, B118, B122, B124 to B126, B128 to B135.

The present invention will be further detailed in the following examples and figures wherein:

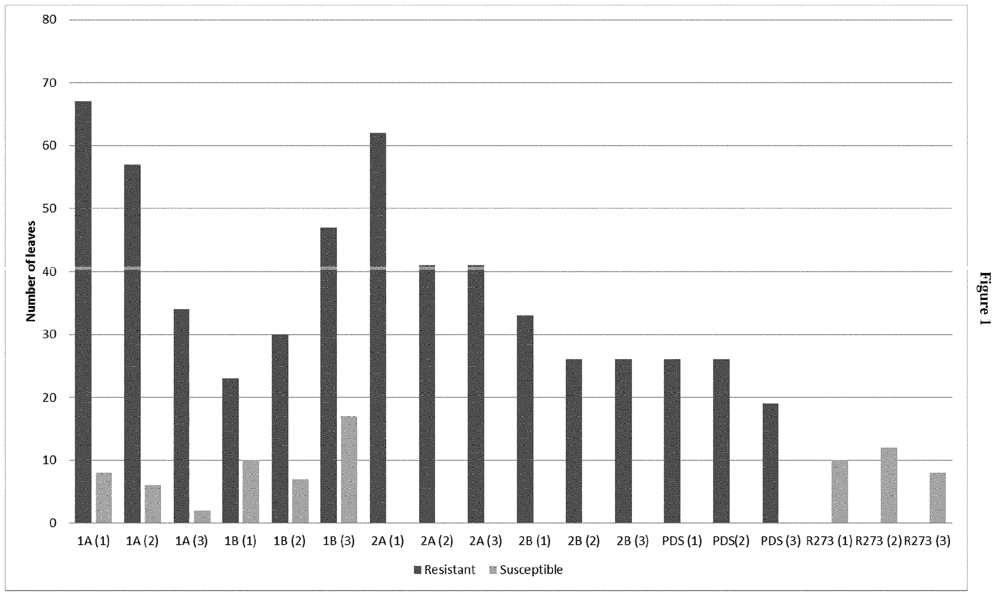

- **Figure 1:**: shows the number (#) of leaves of Lettuce (Y-axis) that are resistant or susceptible to *Bremia lactucae* after VIGS silencing of either *SL7R* (by 1A and 1B), or gene Lsa042767 (by 2A or 2B) (X-axis). The *SL7R* gene has been silenced in these plants using VIGS gene silencing and subsequently infected with *Bremia lactucae* (B130). On the x-axis from left to right: sample leaves of plants in which the *SL7R* gene is silenced using silencing construct 1A or 1B, sample leaves of plants in which gene Lsa042767 is silenced using silencing construct 2A or 2B, sample leaves of plants in which PDS is silenced, sample leaves of plants of susceptible parent R273. In the samples with a resistant phenotype, there is no Bremia present. In the samples with susceptible phenotypes, Bremia is present. As expected with transient gene silencing, VIGS gene silencing does not result in fully 100% silencing of the *SL7R* gene in all plants. However, the leaves from plants wherein the resistance gene has been silenced by VIGS silencing, showed a higher number of susceptible leaves when infected with Bremia as compared to plants where the *SL7R* gene was not silenced. Indeed no susceptible leaves were observed when *SL7R* expression was not affected by VIGS.

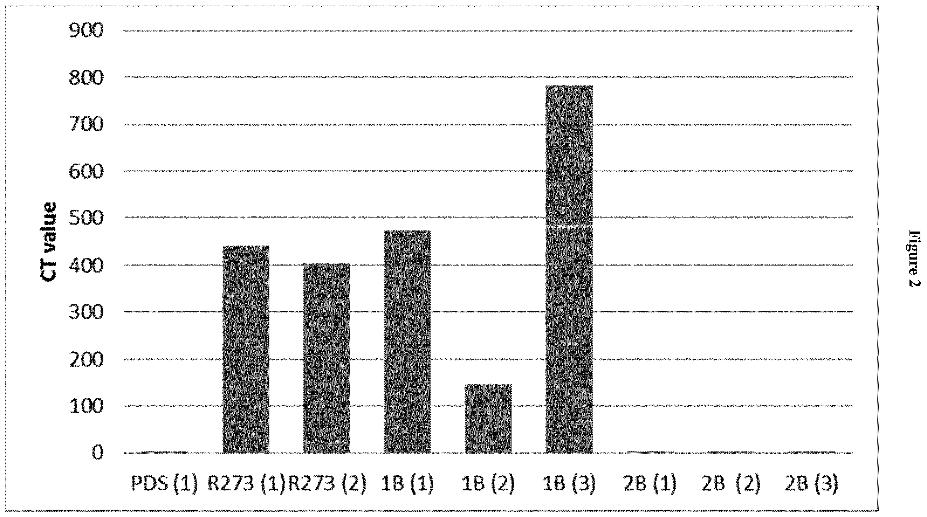

- **Figure 2:**: shows quantification of Bremia actin in Lettuce after VIGS gene silencing. In case *SL7R* gene expression levels were VIGS silenced in lettuce infected with Bremia (B130), expression levels of Bremia actin increased dramatically. The Bremia expression levels in the leaves of plants that showed to be resistant or susceptible to downy mildew after gene silencing, were collected and RNA was isolated to determine the expression levels of Bremia by qPCR. The transcription levels of Bremia lactuca was determined by the transcripts of a Bremia house keeping gene (actin) in relation to the lettuce house keeping gene TUA-3 in a set of leave samples of Lettuce plants of the experiment of Figure 1. Leaves of the plant that were suseptible to *Bremia lactucae* , showed high transcriptional levels of the *Bremia lactucae* house keeping gene actin, indicating the susceptibility corresponds with low *SL7R* gene expression due to VIGS silencing.

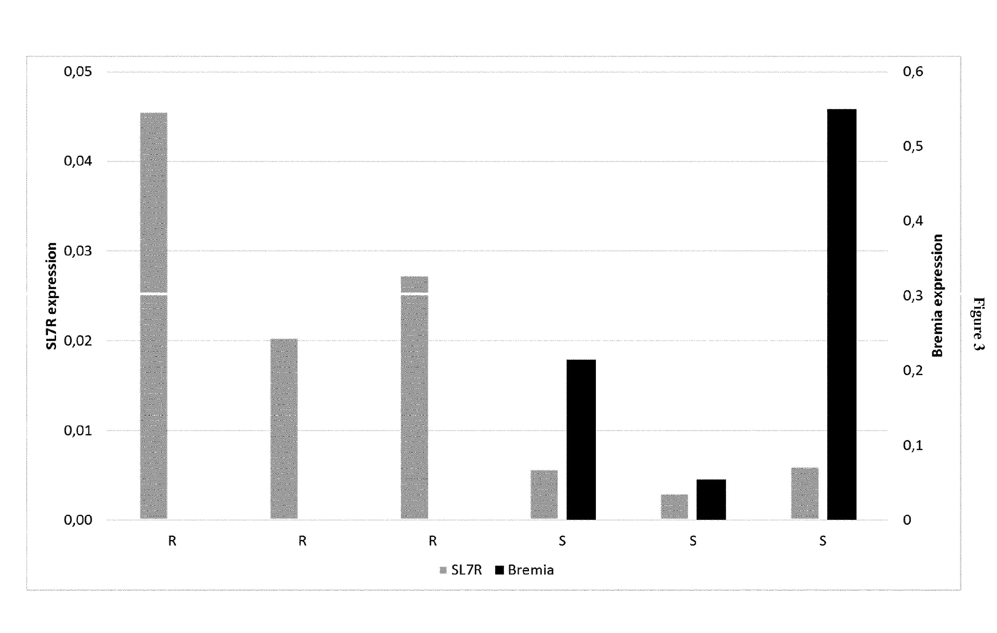

- **Figure 3:**: shows *SL7R* expression levels in *SL7R* VIGS silenced lettuce lines infected with Bremia (B130), determined by qPCR. The transcription levels of *Bremia lactuca* was determined by the transcripts of a Bremia house keeping gene (actin) in relation to the lettuce house keeping gene TUA-3 of *Bremia lactucae* were determined in leave samples of *L. sativa* plants of the experiment of Figure 1. Leaves of the plants that were resistant to *Bremia lactucae* showed to have a high *SL7R* gene expression and low transcriptional levels of the *Bremia lactucae* house keeping gene. Leaves of the plant that were susceptible to *Bremia lactucae,* showed low *SL7R* gene expression (because of VIGS silencing the gene) and high transcriptional levels of the *Bremia lactucae* house keeping gene, indicating the susceptibility corresponds with low *SL7R* gene expression.

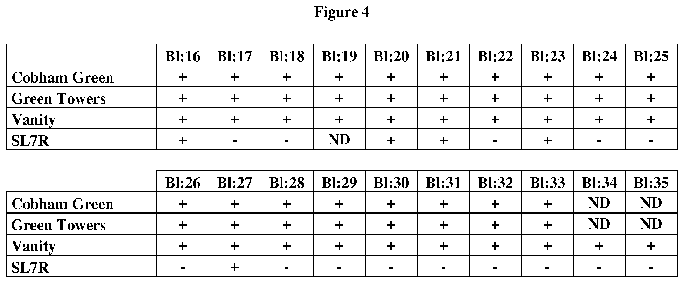

- **Figure 4:**: shows an overview of the disease test performed with the most recent isolates of Bremia B116 to B135 on *L. sativa* lines Cobham Green R273, Green Towers, Vanity and *SL7R.* SL7R is a lettuce plant (*L. sativa*) of present invention comprising the *SL7R* resistance gene. The plant of present invention shows to be resistant to most downy mildew isolates, with the exception of *Bremia lactucae* B116, B120, B121, B123, and B127.

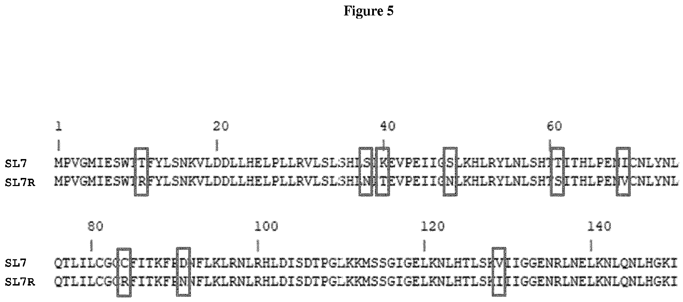

- **Figure 5:**: shows the alignment of the amino acid sequence region of 1 to 150 amino acids, including the LLR region (amino acid 6 to 147) and the single LRR domain (amino acid 26 to 72) of SL7 (SEQ ID No.2) and the SL7R (SEQ ID No.4) protein. The mutations between the two protein sequences have been indicated in the boxed areas. The SL7R protein comprises amino acid substitutions at position 11 (T→R), 38 (S→N), 40 (K→T), 48 (S→N), 61 (T→S), 69 (I→V), 84 (C→R), 91 (D→N) and 129 (V→I).

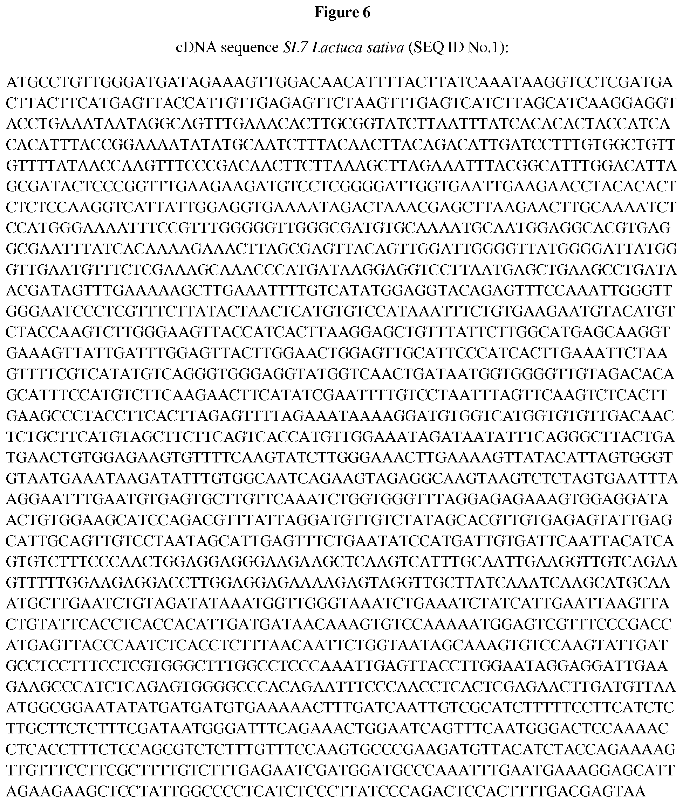

- **Figure 6:**: shows the cDNA sequence (SEQ ID No. 1) encoded by the *SL7* gene of *Lactuca sativa.*

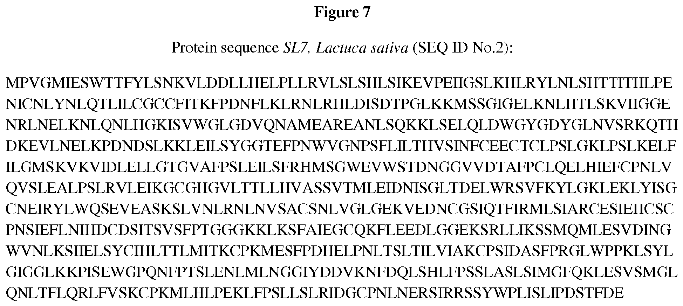

- **Figure 7:**: shows the protein sequence (SEQ ID No. 2) encoded by the *SL7* gene of *Lactuca sativa.*

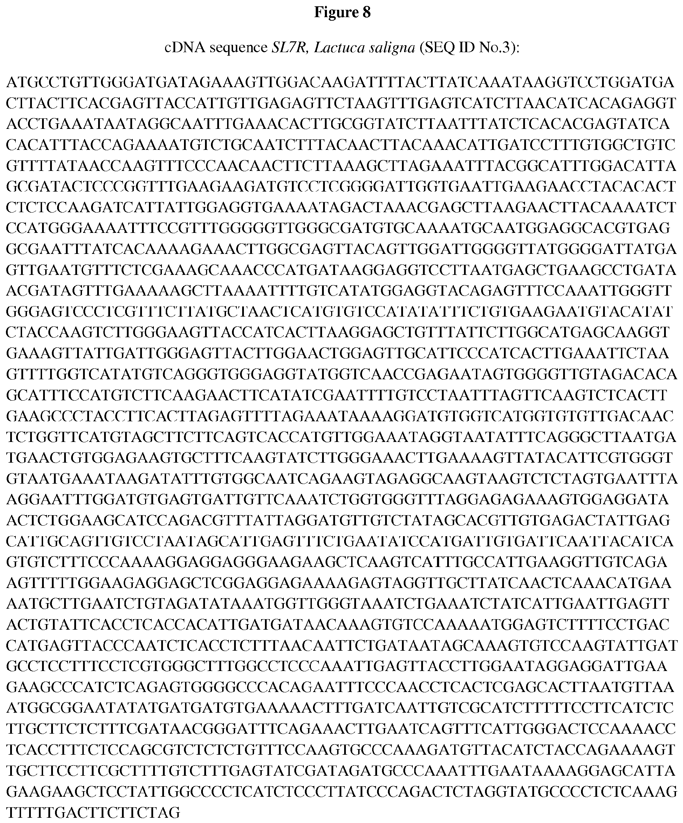

- **Figure 8:**: shows the cDNA sequence (SEQ ID No. 3) encoded by the *SL7R* gene of *Lactuca saligna.*

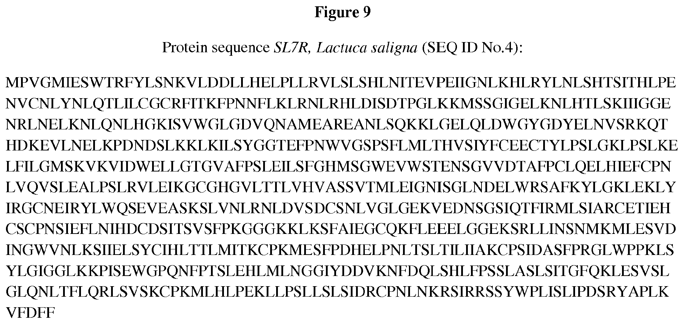

- **Figure 9:**: shows the protein sequence (SEQ ID No. 4) encoded by the *SL7R* gene of *Lactuca saligna.*

### Examples

### Gene Mapping SL7 resistance gene in L. saligna

Gene mapping experiments were done to identify the of the Bremia (*Bremia lactucae*) resistance gene *SL7R* from *Lactuca saligna. SL7R* was originally isolated from *L. saligna* lettuce accession LAC0364. A dominant resistance gene was mapped on chromosome 3, Lettuce genome V8. The *SL7R* resistance gene is the first Bremia resistance gene described and mapped on chromosome 3 in Lettuce.

After fine mapping 12000 plants two putative genes were present. The *SL7R* region is currently flanked by two markers based on a SNP at position V8_3_200695773 and a SNP at position V8_3_200770104. The two candidate genes are designates as Lsa011563 and Lsa042767. VIGS silencing was used to silence both genes independently in a resistant source (*L*. *saligna*), see below. These experiments indicated that when resistance gene Lsa011563 was silenced the plants became susceptible after Bremia infection, whereas when gene Lsa042767 was silenced, the plants remained resistant. This confirms that the Lsa011563 gene provide the plant resistance against Bremia. This resistance gene is renamed to the resistance gene *SL7R* of present invention.

### Construction of SL7 construct and transformation into lettuce (L. saligna).

After gene mapping two candidate resistance genes were found and designated as Lsa011563 (=SL7R) and Lsa042767. To identify which gene (or both) are responsible for the observed resistance, VIGS silencing can be used to silence both genes independently in the resistant source *L. saligna.* Therefore, two VIGS-constructs were made per gene, for Lsa011563 (=SL7) (1A and 1B) and for Lsa042767 (2A and 2B) and cloned in the K20 vector (See Table 1 for sequences, respectively SEQ ID No. 5, SEQ ID No. 6, SEQ ID No. 7, SEQ ID No. 8). Thus two *SL7R* specific VIGS constructs were made (1A and 1B) and two construct that target a different gene Lsa042767 (2A, 2B) can be used as a negative control. The multiple constructs of above were transformed into lettuce (*L. sativa*) using co-cultivation with agrobacterium (GV3101) to study the *SL7R* function. The two resistance candidate genes are individual silenced in VIGS independent experiments. With the leaves of VIGS-experiments independent disease tests (see below) were performed to observe that when *SL7R* was silenced, plants became susceptible to Bremia.

**Table 1.**

| **VIGS-constructs** | **Sequence** |
|---|---|
| Lsa011563_1A | TTCATGTAGCTTCTTCAGTCACCATGTTGGAAATAGGTAATATTT CAGGGCTTAATGATGAACTGTGGAGAAGTGCTTTCAAGTATCTT GGGAAACTTGAAAAGTTATACATTCGTGGGTGTAATGAAATAAG ATATTTGTGGCAATCAGAAGTAGAGGCAAGTAAGTCTCTAGTGA ATTTAAGGAATTTGGATGTGAGTGATTGTTCAAATCTGGTGGGT TTAGGAGAGAAAGTGGAGGATAACTCTGGAAGCATCCAGACGT TTATTAGGATGTTGTCTATAGCACGTTGTGAGA |
| Lsa011563_1B | TGAGTTACCTTGGAATAGGAGGATTGAAGAAGCCCATCTCAGAG TGGGGCCCACAGAATTTCCCAACCTCACTCGAGCACTTAATGTT AAATGGCGGAATATATGATGATGTGAAAAACTTTGATCAATTGT CGCATCTTTTTCCTTCATCTCTTGCTTCTCTTTCGATAACGGGATT TCAGAAACTTGAATCAGTTTCATTGGGACTCCAAAACCTCACCT TTCTCCAGCGTCTCTCTGTTTCCAAGTGCCCAAAGATGTTACATC |
| | TACCAGAAAAGTTGCTTCCTTCGCTTTTGTCTTTGAG |
| Lsa042767_2A | GTGGAGATCAAGCTGGATTATAAGAAGGATTTGTTTGATGGGAA GAGGAATATTGTCACGGCGGAGGAGATAGAGAGCGGGATAAGG CGGCTGATGGAGGATGACGATGTAAGAGAAAAGATAAAAGAGA TGGGGAAAAAGAGCAAAGCGACTGTTAAAGAGGGAGGTTCGTC TTACGCTTCT |
| Lsa042767_2B | CACATTCTTGGAATTAGAAACACGCCCAATCGAGTCGTTGTCTA CCGACAGCAGGATACCGTCTGTGTATCCGGTAGGACCTGTACTG AACCTAGAAGACGGTGCCGGAACACCGCCGGAAAGTGACGTCA TCAGCTGGTTGGACAATCAACCACCTTCCTCGGTTGTTTTCTTGT GTTTTGGGAGTCTGGGATGTTTTGATGAAGTCCAAGTGAAGGAG ATTGCATATGCTTTAGAGCGAAGCGGGCGTTCTTTCTTGTGGTCA CTAC |

### SL7R gene silencing experiment using Virus Induced Gene Silencing (VIGS)

Tobacco rattle virus (TRV)-derived VIGS vectors have been abundantly described to study gene function in *Arabidopsis thaliana, Nicotiana benthamiana, Solanum esculentum* and other plants (see for example Huang C, Qian Y, Li Z, Zhou X.: Virus-induced gene silencing and its application in plant functional genomics. Sci China Life Sci. 2012;55(2):99-108).

Briefly, Lettuce containing *SL7* were silenced for *SL7R* by VIGS. Independent of *SL7R* silencing the *PDS* gene is silenced as well that serves as positive control to indicate if VIGS is working and to determine the efficiency. *PDS* is involved in carotenoid biosynthesis and is the first step in lycopene biosynthesis. This step is catalyzed by phytoene desaturase (PDS). When silencing of the *PDS* gene is achieved, this results in bleached leaves.

Furthermore, all plants that were SL7R-VIGS inoculated were harvested and put in a tray and sprayed with Bremia to test the effect of the gene silencing on disease resistance.

### Disease test and biotest for downy mildew in Lettuce

Leaves of resistant plants transiently transformed with the above described VIGS constructs (1A, 1B, 2A, 2B and PDS), were put in trays with moistened paperboard and infected with Bremia race 30. The infected seedlings are suspended in 20 mL water, filtered by cheesecloth and the flow-through is collected in a spray flask. One tray is spray-inoculated with the *Bremia lactucae* suspension. The trays are covered with a glass plate and stored in a climate chamber at 15°C (12 hours of light). A black, opaque foil is placed over the trays for one day to improve growth of *B. lactucae.* After one day, the foil is removed. Experiments were performed in triple, and eight to ten days after infection leaves are phenotypically scored by eye on the presence of Bremia, i.e. being susceptible or resistant (Figure 1).

Disease resistance tests show that resistance gene *SL7R* provides resistance to most Bremia races from B1:15 to B1:35, with the exceptions being B1:16, BL20, B121, B123, and B1:27. Furthermore a qPCR was performed to determine *SL7R* expression.

A single gene line comprising the SL7R gene used internally to test Bremia diagnostic samples is R290. Seeds of this line are deposited at NCIMB (NCIMB Limited, Ferguson Building; Craibstone Estate, Bucksburn ABERDEEN, Scotland, AB21 9YA United Kingdom) on 12 July 2017 under the number NCIMB 42785.

### Determine Bremia expression in lettuce comprising the SL7R gene

A number of gene expression experiments were conducted in lettuce tissues obtained from the VIGS experiment as outlined above, to determine *SL7R* expression. The response of lettuce leaves to *Bremia lactucae* infection was examined and gene expression studies were used to assess VIGS analysis.

To obtain more insight in the response of lettuce to infection with Bremia, leaves of resistant and susceptible plants were harvested. cDNA was synthesized from RNA that had been isolated from infected leaves. The expression of *SL7R* was assessed in lettuce by conducting qPCR. Expression of *Bremia lactucae* actin and expression of *SL7R* were analyzed by qPCR using the primers as set out in Table 2. (SEQ ID No.9, SEQ ID No.10, SEQ ID No.11, SEQ ID No.12, SEQ ID No.13, and SEQ ID No.14, respectively).

**Table 2.**

| **Primer name** | **Sequence** |
|---|---|
| SL7QPCR-F | 5'-TCCAAGTATTGATGCCTCCTT-3' |
| SL7QPCR-R | 5'-CACTCTGAGATGGGCTTCTTC-3' |
| *B. lactucae* actin Fwd | 5'-GCGAGAAATTGTGCGTGATA-3' |
| *B. lactucae* actin Rv | 5'-ACTCGGCTGCAGTCTTCATT-3' |
| LsTUA-3F | 5'-CTTCTTAGTGTTCAATGCTGTTGG-3' |
| LsTUA-3R | 5'- GAAGGGTAGATAGTGAAACCGAGC-3' |

Figure 2 shows the results of a qPCR of housekeeping gene Bremia actin. Values on the y-axis are CT values (the fold increase is calculated as 2^ -(Ct Bremia actin - Ct TUA3A). On the x-axis from left to right: sample leaf of a plant in which PDS is silenced, sample leaves of plants of susceptible parent R273, sample leaves of plants in which the *SL7R* gene is silenced using silencing construct 1B, sample leaves of plants in which the *SL7R* gene is silenced using silencing construct 2B. In the samples with a resistant (R) phenotype, there is no or almost no Bremia present. In the samples with susceptible (S) phenotypes, high transcription levels of the housekeeping gene Bremia actin were measured.

In addition, Figure 3 shows that in leaves of the plants that are resistant to Bremia, little to no Bremia was detected and that the level of *SL7R* expression was very high. The leaves that originate from plants that are susceptible to Bremia, showed the opposite pattern, a high level of Bremia and low levels of *SL7R* expression.

## Claims

1. A lettuce plant that is resistant to downy mildew, wherein said plant comprises one or more mutations in a *SL7* gene, wherein said *SL7* gene encodes for a protein sequence of SEQ ID No. 2 or having at least 90% sequence identity with SEQ ID No. 2, wherein the one or more mutations in the *SL7* gene comprises an amino acid substitution at position 38 in the SL7 protein represented by SEQ ID No.2, preferably S38N, wherein said plant is not exclusively obtained by means of an essentially biological process.

2. Lettuce plant according to claim 1, wherein the one or more mutations in the *SL7* gene result in amino acid substitutions in the region comprised of amino acid positions 6 to 142 in the SL7 protein represented by SEQ ID No.2.

3. Lettuce plant according to claim 1 or 2, wherein the one or more mutations in the SL7 gene further comprises amino acid substitutions selected from the group consisting of: an amino acid substitution on position 11 in the SL7 protein represented by SEQ ID No.2, preferably T11R; an amino acid substitution at position 40 in the SL7 protein represented by SEQ ID No.2, preferably K40T; an amino acid substitution at position 48 in the SL7 protein represented by SEQ ID No.2, preferably S48N; an amino acid substitution at position 61 in the SL7 protein represented by SEQ ID No.2, preferably T61S; an amino acid substitution at position 69 in the SL7 protein represented by SEQ ID No.2, preferably I69V; an amino acid substitution at position 84 in the SL7 protein represented by SEQ ID No.2, preferably C84R; an amino acid substitution at position 91 in the SL7 protein represented by SEQ ID No.2, preferably D91N; an amino acid substitution at position 129 in the SL7 protein represented by SEQ ID No.2, preferably V129I; amino acid substitutions at position 11, 40, and 84 in the SL7 protein represented by SEQ ID No.2, preferably the mutations are T11R, K40T, and C84R; amino acid substitutions at positions 11, 38, 40, 48, 61, 69, 84, 91 and 129 in the SL7 protein represented by SEQ ID No.2, preferably T11R, S38N, K40T, S48N, T61S, I69V, C84R, D91N, and V129I respectively.

4. Lettuce plant according to any one of the claims 1 to 3, wherein the SL7 gene that comprises one or more mutations encodes for the protein sequence represented by SEQ ID No. 4.

5. Lettuce plant according to any one of the claims 1 to 4, wherein the lettuce plant is selected from *Lactuca sativa, Lactuca virosa, Lactuca saligna, Lactuca serriola, Lactuca aculeate, Lactuca georgica, Lactuca perennis, Lactuca tatarica, Lactuca viminea, preferably Lactuca sativa.*

6. Lettuce plant according to any one of the claims 1 to 5, wherein downy mildew is caused by *Bremia lactucae,* preferably one or more of *Bremia lactucae* selected from the group of race B117, B118, B122, B124 to B126, B128 to B135.

7. Lettuce plant according to any of the claims 1 to 6, wherein the *SL7R* gene of SEQ ID No. 3 is obtainable from deposit number NCIMB 42785.

8. Lettuce plant according to claim 1 to 7, wherein the one or more mutations in the SL7 gene are obtained by mutagenesis or CRISPR/Cas.

9. Seed produced by a lettuce plant according to any one of the claims 1 to 8,
wherein the seed comprises the SL7 gene as defined in said claims, and wherein said seed is not exclusively obtained by means of an essentially biological process.

10. A resistance gene *SL7R* for conferring resistance to *Bremia lactucae* in a lettuce plant, wherein said gene comprises at least 95% sequence identity with the coding sequence of SEQ ID No. 3, wherein the gene encodes for the SL7R protein comprising at least 95% sequence identity with SEQ ID No. 4, wherein said SL7R protein comprises an N on position 38 of SEQ ID No. 4.

11. Resistance gene *SL7R* according to claim 10, wherein said SL7R protein further comprises an R at positions 11, and/or a T at position 40, and/or an N at position 48, and/or an S at position 61, and/or a V at position 69, and/or an R at position 84, and/or an N at position 91 and/or an I at position 129, preferably said SL7R protein comprises all of said amino acids on said positions.

12. Use of a gene construct plasmid for introducing a resistance gene into the genome of a plant or plant cell, wherein the gene construct is comprised of a resistance gene *SL7R* according to claim 10 or claim 11 operably linked to expression providing sequences in said plant.

13. Method for obtaining a lettuce plant that is resistant to downy mildew,
wherein the method comprises the step of providing one or more mutations in a *SL7* gene of a lettuce plant, resulting in a *SL7R* resistance gene according to claim 10 or 11, wherein the mutations in the *SL7* gene are obtained by by mutagenesis or CRISPR/Cas.

14. Method according to claim 13, wherein the one or more mutations in the *SL7* gene result in amino acid substitutions in the region comprised of amino acid positions 6 to 147 in the SL7 protein represented by SEQ ID No.2.

15. Method according to any one of the claims 13 or 14, wherein the one or more mutations in the *SL7* gene comprises amino acid substitutions at position 11, 38, 40, 84 48, 61, 69, 91 and/or 129 in the SL7 protein represented by SEQ ID No.2.

## Patentansprüche

1. Salatpflanze, die beständig gegen Falschen Mehltau ist, wobei die Pflanze eine oder mehrere Mutationen in einem SL7-Gen umfasst, wobei das SL7-Gen für eine Proteinsequenz von SEQ ID No. 2 codiert oder mindestens 90 % Sequenzidentität mit SEQ ID No. 2 aufweist, wobei die eine oder die mehreren Mutationen in dem SL7-Gen eine Aminosäuresubstitution an Position 38 in dem SL7-Protein, dargestellt durch SEQ ID No. 2, vorzugsweise S38N, umfasst, wobei die Pflanze nicht ausschließlich mittels eines im Wesentlichen biologischen Prozesses erhalten wird.

2. Salatpflanze nach Anspruch 1, wobei die eine oder die mehreren Mutationen in dem SL7-Gen zu Aminosäuresubstitutionen in der Region führen, die aus Aminosäurepositionen 6 bis 142 in dem SL7-Protein bestehen, das durch SEQ ID No. 2 dargestellt wird.

3. Salatpflanze nach Anspruch 1 oder 2, wobei die eine oder die mehreren Mutationen in dem SL7-Gen ferner Aminosäuresubstitutionen umfasst, die aus der Gruppe ausgewählt sind, bestehend aus:
eine Aminosäuresubstitution an Position 11 in dem SL7-Protein, das durch SEQ ID No. 2, dargestellt wird, vorzugsweise T11R; eine Aminosäuresubstitution an Position 40 in dem SL7-Protein, das durch SEQ ID No. 2 dargestellt wird, vorzugsweise K40T; eine Aminosäuresubstitution an Position 48 in dem SL7-Protein, das durch SEQ ID No. 2 dargestellt wird, vorzugsweise S48N; eine Aminosäuresubstitution an Position 61 in dem SL7-Protein, das durch SEQ ID No. 2 dargestellt wird, vorzugsweise T61S; eine Aminosäuresubstitution an Position 69 in dem SL7-Protein, das durch SEQ ID No. 2 dargestellt wird, vorzugsweise I69V; eine Aminosäuresubstitution an Position 84 in dem SL7-Protein, das durch SEQ ID No. 2 dargestellt wird, vorzugsweise C84R; eine Aminosäuresubstitution an Position 91 in dem SL7-Protein, das durch SEQ ID No. 2 dargestellt wird, vorzugsweise D91N; eine Aminosäuresubstitution an Position 129 in dem SL7-Protein, das durch SEQ ID No. 2 dargestellt wird, vorzugsweise V129I; Aminosäuresubstitutionen an Position 11, 40 und 84 in dem SL7-Protein, das durch SEQ ID No. 2 dargestellt wird, vorzugsweise die Mutationen T11R, K40T und C84R sind; Aminosäuresubstitutionen an den Positionen 11, 38, 40, 48, 61, 69, 84, 91 und 129 in dem SL7-Protein, das durch SEQ ID No. 2 dargestellt wird, vorzugsweise T11R, S38N, K40T, S48N, T61S, I69V, C84R, D91N beziehungsweise V129I.

4. Salatpflanze nach einem der Ansprüche 1 bis 3, wobei das SL7-Gen, das eine oder mehrere Mutationen umfasst, für die Proteinsequenz codiert, die durch SEQ ID No. 4 dargestellt wird.

5. Salatpflanze nach einem der Ansprüche 1 bis 4, wobei die Salatpflanze aus *Lactuca sativa, Lactuca virosa, Lactuca saligna, Lactuca serriola, Lactuca aculeate, Lactuca georgica, Lactuca perennis, Lactuca tatarica, Lactuca viminea, vorzugsweise Lactuca sativa* ausgewählt ist.

6. Salatpflanze nach einem der Ansprüche 1 bis 5, wobei der Falsche Mehltau durch *Bremia lactucae,* vorzugsweise eine oder mehrere von *Bremia lactucae,* die aus der Gruppe der Rasse Bl17, Bl18, Bl22, BI24 bis Bl26, Bl28 bis BI35 ausgewählt sind, verursacht wird.

7. Salatpflanze nach einem der Ansprüche 1 bis 6, wobei das SL7R-Gen von SEQ ID No. 3 aus einer Hinterlegungsnummer NCIMB 42785 erhältlich ist.

8. Salatpflanze nach Anspruch 1 bis 7, wobei die eine oder mehreren Mutationen im SL7-Gen durch Mutagenese oder CRISPR/Cas erhalten werden.

9. Samen, der von einer Salatpflanze nach einem der Ansprüche 1 bis 8 produziert wird, wobei der Samen das SL7-Gen wie in den Ansprüchen definiert umfasst, und wobei der Samen nicht ausschließlich mittels eines im Wesentlichen biologischen Prozesses erhalten wird.

10. Resistenzgen SL7R für ein Verleihen von Resistenz gegen *Bremia lactucae* in einer Salatpflanze, wobei das Gen mindestens 95 % Sequenzidentität mit der codierenden Sequenz von SEQ ID No. 3 umfasst, wobei das Gen für das SL7R-Protein codiert, umfassend mindestens 95 % Sequenzidentität mit SEQ ID No. 4, wobei das SL7R-Protein ein N an Position 38 von SEQ ID No. 4 umfasst.

11. Resistenzgen SL7R nach Anspruch 10, wobei das SL7R-Protein ferner ein R an Positionen 11 und/oder ein T an Position 40 und/oder ein N an Position 48 und/oder ein S an Position 61 und/oder ein V an Position 69 und/oder ein R an Position 84 und/oder ein N an Position 91 und/oder ein I an Position 129 umfasst, vorzugsweise das SL7R-Protein alle der Aminosäuren an den Positionen umfasst.

12. Verwendung eines Genkonstruktplasmids zum Einführen eines Resistenzgens in das Genom einer Pflanze oder Pflanzenzelle, wobei das Genkonstrukt aus einem Resistenzgen *SL7R* nach Anspruch 10 oder 11 besteht, das mit expressionsbereitstellenden Sequenzen in der Pflanze wirkverknüpft ist.

13. Verfahren zum Erhalten einer Salatpflanze, die beständig gegen Falschen Mehltau ist, wobei das Verfahren den Schritt des Bereitstellens einer oder mehrerer Mutationen in einem *SL7*-Gen einer Salatpflanze umfasst, was zu einem *SL7R*-Resistenzgen nach Anspruch 10 oder 11 führt, wobei die Mutationen in dem *SL7*-Gen durch Mutagenese oder CRISPR/Cas erhalten werden.

14. Verfahren nach Anspruch 13, wobei die eine oder die mehreren Mutationen in dem *SL7*-Gen zu Aminosäuresubstitutionen in der Region führen, die aus den Aminosäurepositionen 6 bis 147 in dem SL7-Protein bestehen, das durch SEQ ID No. 2 dargestellt wird.

15. Verfahren nach einem der Ansprüche 13 oder 14, wobei die eine oder die mehreren Mutationen in dem *SL7*-Gen Aminosäuresubstitutionen an Position 11, 38, 40, 84, 48, 61, 69, 91 und/oder 129 in dem SL7-Protein umfasst, das durch SEQ ID No. 2 dargestellt wird.

## Revendications

1. Plant de laitue qui est résistant au mildiou, dans lequel ledit plant comprend une ou plusieurs mutations dans un gène *SL7,* dans lequel ledit gène *SL7* code pour une séquence de protéine de SEQ ID No. 2 ou ayant au moins 90 % d'identité de séquence avec SEQ ID No. 2, dans lequel la ou les mutations dans le gène *SL7* comprennent une substitution d'acide aminé à la position 38 dans la protéine SL7 représentée par SEQ ID No. 2, de préférence S38N, dans lequel ledit plant n'est pas exclusivement obtenu au moyen d'un processus sensiblement biologique.

2. Plant de laitue selon la revendication 1, dans lequel la ou les mutations dans le gène *SL7* résultent en des substitutions d'acide aminé dans la région constituée des positions d'acide aminé 6 à 142 dans la protéine SL7 représentée par SEQ ID No. 2.

3. Plant de laitue selon la revendication 1 ou 2, dans lequel la ou les mutations dans le gène SL7 comprennent en outre des substitutions d'acide aminé choisies dans le groupe constitué de :
une substitution d'acide aminé à la position 11 dans la protéine SL7 représentée par SEQ ID No. 2, de préférence T11R ; une substitution d'acide aminé à la position 40 dans la protéine SL7 représentée par SEQ ID No. 2, de préférence K40T ; une substitution d'acide aminé à la position 48 dans la protéine SL7 représentée par SEQ ID No. 2, de préférence S48N ; une substitution d'acide aminé à la position 61 dans la protéine SL7 représentée par SEQ ID No. 2, de préférence T61S ; une substitution d'acide aminé à la position 69 dans la protéine SL7 représentée par SEQ ID No. 2, de préférence I69V ; une substitution d'acide aminé à la position 84 dans la protéine SL7 représentée par SEQ ID No. 2, de préférence C84R ; une substitution d'acide aminé à la position 91 dans la protéine SL7 représentée par SEQ ID No. 2, de préférence D91N ; une substitution d'acide aminé à la position 129 dans la protéine SL7 représentée par SEQ ID No. 2, de préférence V129I ; des substitutions d'acide aminé à la position 11, 40 et 84 dans la protéine SL7 représentée par SEQ ID No. 2, de préférence les mutations sont T11R, K40T et C84R ; des substitutions d'acide aminé aux positions 11, 38, 40, 48, 61, 69, 84, 91 et 129 dans la protéine SL7 représentée par SEQ ID No. 2, de préférence T11R, S38N, K40T, S48N, T61S, I69V, C84R, D91N et V129I respectivement.

4. Plant de laitue selon l'une quelconque des revendications 1 à 3, dans lequel le gène SL7 qui comprend une ou plusieurs mutations code pour la séquence de protéine représentée par SEQ ID No. 4.

5. Plant de laitue selon l'une quelconque des revendications 1 à 4, dans lequel le plant de laitue est choisi parmi *Lactuca sativa, Lactuca virosa, Lactuca saligna, Lactuca serriola, Lactuca aculeate, Lactuca georgica, Lactuca perennis, Lactuca tatarica, Lactuca viminea, de préférence Lactuca sativa.*

6. Plant de laitue selon l'une quelconque des revendications 1 à 5, dans lequel le mildiou est causé par *Bremia lactucae,* de préférence un ou plusieurs parmi des *Bremia lactucae* choisis dans le groupe de races B117, B118, B122, B124 à B126, B128 à B135.

7. Plant de laitue selon l'une quelconque des revendications 1 à 6, dans lequel le gène *SL7R* de SEQ ID No. 3 peut être obtenu du numéro de dépôt NCIMB 42785.

8. Plant de laitue selon la revendication 1 à 7, dans lequel la ou les mutations dans le gène SL7 sont obtenues par mutagenèse ou CRISPR/Cas.

9. Semence produite par un plant de laitue selon l'une quelconque des revendications 1 à 8, dans laquelle la semence comprend le gène SL7 tel que défini dans lesdites revendications, et dans laquelle ladite semence n'est pas exclusivement obtenue au moyen d'un processus sensiblement biologique.

10. Gène de résistance *SL7R* permettant de conférer une résistance à *Bremia lactucae* dans un plant de laitue, dans lequel ledit gène comprend au moins 95 % d'identité de séquence avec la séquence codante de SEQ ID No. 3, dans lequel le gène code pour la protéine SL7R comprenant au moins 95 % d'identité de séquence avec SEQ ID No. 4, dans lequel ladite protéine SL7R comprend un N à la position 38 de SEQ ID No. 4.

11. Gène de résistance *SL7R* selon la revendication 10, dans lequel ladite protéine SL7R comprend en outre un R aux positions 11, et/ou un T à la position 40, et/ou un N à la position 48, et/ou un S à la position 61, et/ou un V à la position 69, et/ou un R à la position 84, et/ou un N à la position 91 et/ou un 1 à la position 129, de préférence ladite protéine SL7R comprend la totalité desdits acides aminés auxdites positions.

12. Utilisation d'un plasmide de construction génique permettant d'introduire un gène de résistance dans le génome d'un plant ou d'une cellule de plant, dans lequel la construction génique est constituée d'un gène de résistance *SL7R* selon la revendication 10 ou la revendication 11 lié fonctionnellement à des séquences de fourniture d'expression dans ledit plant.

13. Procédé permettant d'obtenir un plant de laitue qui est résistant au mildiou, dans lequel le procédé comprend l'étape de fourniture d'une ou plusieurs mutations dans un gène *SL7* d'un plant de laitue, résultant en un gène de résistance *SL7R* selon la revendication 10 ou 11, dans lequel les mutations dans le gène *SL7* sont obtenues par mutagenèse ou CRISPR/Cas.

14. Procédé selon la revendication 13, dans lequel la ou les mutations dans le gène *SL7* résultent en des substitutions d'acide aminé dans la région constituée des positions d'acide aminé 6 à 147 dans la protéine SL7 représentée par SEQ ID No. 2.

15. Procédé selon l'une quelconque des revendications 13 ou 14, dans lequel la ou les mutations dans le gène *SL7* comprennent des substitutions d'acide aminé à la position 11, 38, 40, 84, 48, 61, 69, 91 et/ou 129 dans la protéine SL7 représentée par SEQ ID No. 2.
